(19) Europäisches Patentamt
European Patent Office
Office européen des brevets

(11) Publication number: **0 343 307 A1**

# EUROPEAN PATENT APPLICATION

(21) Application number: **88500050.5**

(22) Date of filing: **26.05.88**

(51) Int. Cl.⁴: **C07D 211/58 , C07D 211/26 , C07D 409/12 , C07D 417/12 , C07D 401/12 , C07D 405/12 , C07D 409/14 , C07D 417/14 , C07D 401/14 , C07D 405/14 , //A61K31/445,A61K31/44**

Amended claims in accordance with Rule 86 (2) EPC.

(43) Date of publication of application: **29.11.89 Bulletin 89/48**

(84) Designated Contracting States: **BE CH DE FR GB IT LI LU NL SE**

(71) Applicant: **FABRICA ESPANOLA DE PRODUCTOS QUIMICOS Y FARMACEUTICOS, S.A.**

**Leioa-Lamiaco Vizcaya(ES)**

(72) Inventor: **Orjales Venero, Aurelio**
**Paseo del Puerto, 24**
**E-48990 Neguri (Vizcaya)(ES)**
Inventor: **Toledo Avello, Antonio**
**Amesti, 15, 2o**
**E-48900 Algorta (Vizcaya)(ES)**

(74) Representative: **Isern-Cuyas, Maria Luisa**
**Paseo de la Castellana 131, Bajo C**
**E-28046 Madrid(ES)**

(54) **4-Piperidinealkanamine derivatives.**

㊼ Compounds of general formula I (Z=O) are prepared by reacting 4-piperidinealkanamines with carboxylic acids or reactive derivatives thereof, such as the esters of lower aliphatic alcohols or acid halides. Compounds of general formula I (Z=CONH) are likewise obtained by means of the reaction between 4-piperidinealcanamines and isocyanates.

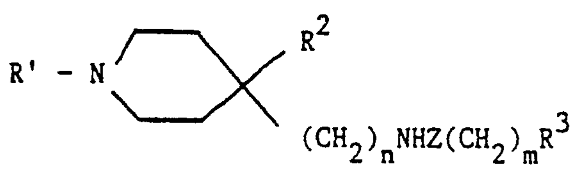

(I)

The compounds of general formula I thus obtained, and the pharmaceutically acceptable salts thereof are of a great therapeutic utility, given their activity as specific antagonists of histamine $H_1$ receptors.

EP 0 343 307 A1

# NEW 4-PIPERIDINEALCANAMINE DERIVATIVES

## INTRODUCTION

Current availability of $H_1$ antihistamines without central effects has afforded great therapeutic progress, and has again promoted an increasingly greater interest in the development of new structures having this property. The enormous differences which exist as regards chemical structure, specific action, etc., in non-sedative $H_1$ antagonists render the search for structure-activity relationship difficult. It seems that, up to now, the fact that a molecule should have these two properties, both an antihistamine action and the inability to cross the hematoencephalic barrier, this latter property depending on its liposolubility, is purely a matter of chance.

Moreover, most classical $H_1$ antihistamines likewise present a serotonine antagonism, anticholinergic activity and a blocking of the $alpha_1$ adrenergic receptors, responsible for several side-effects linked to the main activity, which limits its own therapeutic action.

For this reason, the search for new antihistamine molecules is centered on the synthesis of a pure $H_1$ antagonist having a greater affinity for peripheral rather than central histamine receptors -this property being intensified if the drug has difficulty to cross the hematoencephalic barrier - and a small or null antagonist activity towards other biological amines.

Some 4-piperidinealcanamine derivatives have a marked activity as specific antagonists of the histamine $H_1$ receptors.

The present invention relates to the preparation of new 4-piperidinealcanamine derivatives which may be used successfully in the treatment of allergies.

It consists in reacting a reactive derivative of an alkylarylic alkylheteroarylic or heteroarylic carboxylic acid, or the carboxylic acid itself, with a 4-piperidinealcanamine to form a carboxamide of general formula I (Z = CO) and the pharmacologically acceptable salts thereof.

When 4-piperidinalcanamine is reacted with an isocyanate, $R^3(CH_2)_mNCO$, a urea derivative of general formula I (Z = CONH), is formed.

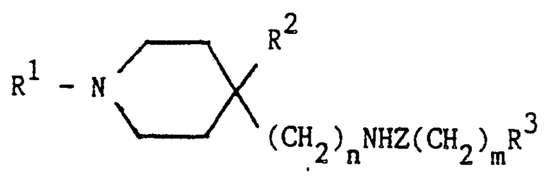

I

The meanings of general formula I are as follows:

$R^1$ = alkylic ($C_1$ - $C_4$), alicyclic ($C_5$ - $C_6$), alkylaylic or alkylheteroarylic organic radical, such as, for example, methyl, ethyl, isopropyl, cyclopentyl, cyclohexyl, 2-phenylethyl, phenylmethyl, 2-piperidinylmethyl, 3-piperidinylmethyl, 4-piperidinylmethyl, 4-phenyl-4-hydroxybutyl, etc., and the corresponding radicals substituted in the aromatic ring by $CH_3$, $C_2H_5$, $OH.OCH_3$, $CF_3$, F, Cl, Br, I, $NH_2$ and $NO_2$.

Z = carbonyl (CO) or carbonylamine (CONH) group.

$R^2$ = hydroxyl group (OH) or a hydrogen atom (H).

n = either 0, 1 or 2.

m = either 0, 1 or 2.

$R^3$ = arylic, alkylarylic, alkylheteroarylic or heteroarylic radical, which may in turn be substituted within the aromatic ring by $CH_3$, $C_2H_5$, $OCH_3$, $OCF_3$, OH, F, Cl, Br, I, $NH_2$, $NR^4R^5$, $COOH.OCOCH_3$, $NO_2$($R^4R^5$ = acetyl, sulfonyl, $CH_3$, H).

Thus there is obtained, for example: 4-chlorophenylmethyl, 4-methylphenylmethyl, 4-hydroxyphenylmethyl, 4-fluorophenylmethyl, 4-acetyloxyphenylmethyl, 4-methoxyphenylmethyl, 3-chlorophenylmethyl, 3-methylphenylmethyl, 3-hydroxyphenylmethyl, 3-fluorophenylmethyl, 3-acetyloxyphenylmethyl, 3-methoxyphenylmethyl, 2-chlorophenylmethyl, 2-methylphenylmethyl, 2-hydroxyphenylmethyl, 2-fluorophenylmethyl, 2-acetyloxyphenylmethyl, 2-methoxyphenylmethyl, 2-thiophenemethyl, 3-thiophenemethyl, 2-thiophene-ethenyl, 2-(3-thiophenyl)-ethenyl, 2-furanomethyl, 3-furanomethyl, 2-pyridyl-methyl, 3-pyridylmethyl, 4-pyridyl-methyl, 2-phenyl-ethyl, 2-thiophenyl, 3-thiophenyl, 2-furanyl, 3-furanyl, 2-pyridyl, 3-pyridyl, 4-pyridyl, 4-hydroxyphenyl 2-thiazolyl, 5-thiazolyl, 2-(3-methyl)-thiophenyl, 2-(5-methyl)-thiophenyl, 2-(3-methoxy-5-methyl)-thiophenyl, 2-(3-methoxy-5-phenyl)-thiophenyl, 2-(3-methylsulfonylamine)-thiophenyl, 2-(3-methylsulfonylaminomethyl)-thiophenyl, 5-(2,4-dimethyl)-thiazolyl, etc.

$R^3$ can also mean a cyclic or heterocyclic carbon radical, with more or less unsaturation, such as, for example: cyclohexyl, 1-cyclohexenyl cyclopentyl, 1-cyclopentenyl, 2-tetrahydrothiophenyl, 2-tetrahydrothiophenyl-methyl, 2-tetrahydrofuranyl, etc.

The 4-piperidinalcanamines used in the present invention are compounds which are sufficiently described in the literature. Some of them, such as 1-(1-methylethyl)-4-piperidinamine, 1-methyl-4-hydroxy-4-aminomethyl-piperidine, 1-(3-pyridyl-methyl)-4-aminopiperidine, etc. are not described in the literature, but they may be prepared, with very high yields by the methods so described in the bibliography, such as, for example, N.H. Harper et al. (J. Med. Chem, 7(6), 729 (1964)), or G. Regnier (Chim. Ther. 3, 185 (1969)), from easily obtained products, such as ethyl acrylate, isopropylamine, methylamine, 3-aminomethyl-piridine, etc., which are transformed into the corresponding N-substituted 4-piperidinones. The derivatives of 4-piperidinamines ($R^2$ = H) are obtained by reduction of its oximes. The reaction between the 4-piperidones and nitro-methane, which process is likewise described in the bibliography. G. Regnier (Chim. Ther. 174 (1969)) gives 4-hydroxy-4-nitromethyl-piperidines, which, on being reduced by $Zn/H^+$ (Belg. 818.471) produce 4-hydroxy-4-aminomethylpiperidine ($R^2$ = OH) derivatives.

The reduction of 4-piperidinones with $NaBH_4$ and subsequent treatment of the 4-piperidinol obtained with p-toluensulfonyl and KOH results in 4-cyanopiperidines which are reduced with LiAlH and to the corresponding 4-piperidine-methanamines, such as, for example, 1-(1-methyl ethyl)-4-piperidine-methanamine or 1-(2-phenylethyl)-4-piperidine-methanamine.

As regards the carboxylic acids used, and their reactive derivatives, these are products described in the bibliography. Thus, synthesis of 2-tetrahydrothiophenyl-acetic acid is effected by the method of Wrobel et al. (Synthesis, 452 (1987)) for obtaining 2-tetrahydrothiofene-carboxylic acid, and the subsequent Arndt-Eistart reaction to which it is subjected.

The aromatic or alkylarylic isocyanates used in the preparation of compounds of formula I (Z = CONH) are obtained by means of the process described by H. Ulrich et al. (Synthesis, 277 (1979).

The invention is performed, in practice, by reacting a carboxylic acid $R^3(CH_2)_mCOOH$ with a 4-piperidinalcanamine in the presence of a condensing agent such as N,N′-dicyclohexylcarbodiimide in a suitable inert solvent, such as tetrahydrofurane, dioxane, dimethylformamide or dimethylsulfoxide, at temperatures of between 20 and 60° C and in a period of from 1 to 24 hours.

The process can also be effected by reacting an acid chloride $R^3(CH_2)_mCOCl$ with a suitable 4-piperidinalcanamine in ethyl ether, chloroform, tetrahydrofurane, dichloromethane, dioxane or benzene at 20 to 60° C for 1 to 5 hours, to give the carboxamide chlorhidrate of general formula I.

A further variant of the invention consists in using an ester $R^3(CH_2)_mCOOR^6$ ($R^6$ = Me, Et, $MaOCH_2CH_2$, which is reacted with the 4-piperidinalcanamine in a solvent having a high boiling point, such as toluene, xylene, dimethylformamide, etc., at the boiling temperature thereof during 6 to 48 hours in the presence of suitable condensing agents, such as, for example, 4A molecular sieve, sodium ethoxide or 4-methyl-benzenesulfonic acid. The carboxamide formed is purified by crystallization in a suitable solvent, usually mixtures of EtOH, MeOH or isopropanol with $H_2O$.

Finally, the reaction between isocyanates $R^3(CH_2)_mNCO$ and 4-piperidinalcanamines takes place in an inert solvent such as ethyl acetate, toluene, ethyl ether or tetrahydrofurane, at temperatures of between 0 and 20° C and for a period of time of from 1 to 24 hours.

## EXAMPLES

The following examples serve exclusively to illustrate the present invention, wherefore all details not affecting the contents thereof may be modified, and in no case may they be considered as limiting the invention in any way.

Example 1. N- [-(2-PHENYLETHYL)-4-HYDROXY-4-PIPERIDINYL] METHYL-2-THIOPHENECARBOXAMIDE CHLORHIDRATE

A solution of 12 g of 4-hydroxy-4-aminomethyl-1-(2-phenylethyl)piperidine in 200 ml of tetrahydrofurane is added to a vigorously-shaken mixture of 10 g of 2-thiophenocarboxylic acid and 16 g of N,N-dicyclohexylcarbodiimide in 100 ml of tetrahydrofurane at 20 to 25° C. After 24 hours the reacting mixture is filtered, and the filtrate is concentrated to dryness at reduced pressure. The residue is dissolved in 50 ml

of ethanol and the solution is subjected to a stream of dry HCl until cloudiness starts to appear, thus crystallizing the carboxamide chlorhydrate formed (Yield 65 - 70 %). MP: 255-7 ° C (d).

Example 2. N- [1-(2-PHENYLETHYL)-4-PIPERIDINYL] -2-THIOPHENECARBOXAMIDE

50 g of 1-(2-phenylethyl)-4-aminopiperidine chlorhydrate are shaken with 500 ml of a 2N KOH solution, externally cooling in a water bath. 150 ml of toluene and 25 ml of the chloride of 2-thiophenecarboxylic acid are added, shaking vigorously. A white carboxamide precipitate is formed.

After 3 hours at 20° C the reacting mixture is filtered, and 95% of the desired product is obtained, which can be recrystallized from ethanol-water. 187-190° C (base).

Example 3. 2,4-DIMETHYL-N- [1-(2-PHENYLETHYL)-4-HYDROXY-4-PIPERIDINYL] METHYL-1,3-THIAZOLE-5-CARBOXAMIDE DICHLORHYDRATE

A solution of 1,23 g of the chlorhydrate of 2,4-dimethyl-1,3-thiazole-5-carboxylic acid in 20 ml of ethyl ether is added to a suspension of 1,2 g of 1-(2-phenylethyl)-4-hydroxy-4-aminomethyl-piperidine in 40 ml of ethyl ether, vigorously shaken. A light-coloured precipitate is rapidly formed. After 1 hour at 20° C it is filtered and stored in a vaccuum drier. It is an extremely hygroscopic product, deliquescent, with MP = 300° C (yield: 80%).

Example 4. N- [1-(METHYLETHYL)-4-PIPERIDINYL] -2-THIOPHENECARBOXAMIDE

A solution of ethyl 2-thiophenecarboxylate (15.6 g) in 200 ml of anhydrous xylene is treated with 13 g of 1-(1-methylethyl)-4-aminopiperidine, and the mixture is heated to reflux for 8 hours in a Soxhlet-type extraction system, the cartridge whereof contains 10 g of 4A molecular sieves. The reacting mixture is concentrated under low pressure, and the residue is treated with 100 ml of ethanol and then subjected to a stream of dry HCl gas until it reaches a pH of 5. The chlorhydrate of the carboxamide is crystallized on standing. Yield 65%. (MP: 150 - 152° C).

Example 5. N- [1-(2-PHENYLETHYL)-4-HYDROXY-4-PIPERIDINYL] METHYL-2-THIOPHENEPROPENAMIDE

6.2 g of 2-thiophenepropanoic acid are heated with 5 g of $SOCl_2$ up to 140° C until gases are not evolved (1 hour), the crude product is dissolved in 100 ml of anhydrous THF, and this solution added, dropwise, onto a well-shaken and cooled solution of 8 g of 1-(2-phenylethyl)-4-aminomethylpiperidine in 250 ml of anhydrous THF. After shaking the resulting mixture for 5 hours at 20° C, the precipitate obtained, namely the carboxamide chlorhydrate of 2-thiophenepropanoic acid, is collected. Yield: 85%. MP: 245 - 247° C.

Example 6. N- [1-(2-PHENYLETHYL)-4-PIPERIDINYL] -TETRAHYDRO-2-THIOPHENECARBOXAMIDE

In accordance with the process described in Example 5, and using 2-tetrahydrothiophenecarboxylic acid and 1-(2-phenylethyl)-4-piperidinamine, the corresponding carboxamide is obtained. Yield: 85%. (MP: 258 - 260° C).

Example 7. 4-FLUORO-N- [1-(2-PHENYLETHYL)-4-PIPERIDINYL] -BENZENEACETAMIDE

By treatment of 1-(2-phenylethyl)-4-aminopiperidine in $Et_2O$ with the chloride of 4-fluorobenzeneacetic acid under the conditions described in Example 5, the corresponding carboxamide chlorhydrate is obtained, in the form of a white, hygroscopic solid. 4-fluoro-N-1-(2-phenylethyl)-4-piperidinyl-benzeneacetamide in the form of a base is obtained by dissolving the white solid in $H_2O$ and adjusting the pH of the solution to 8.5 using NaOH. Yield: 75%. (MP: 160-163° C).

Example 8. N- [1-(2-PHENYLETHYL-4-PIPERIDINYL] -4-PYRIDINCARBOXAMIDE

Using the same process as described in Example 5, the chlorhydrate of isonicotinic acid chloride is obtained, which, upon treatment with 1-(2-phenylethyl)-4-piperidineamine in anhydrous THF, gives the dichlorhydrate of the corresponding carboxamide. White solid, extremely hygroscopic. Yield: 55% (MP: 260° C).

Example 9. 3-METHYL-N- [1-(2-PHENYLETHYL)-4-PIPERIDINYL] METHYL-2-THIOPHENECARBOXAMIDE

0.5 moles of 3-methyl-2-thiophenecarboxylic acid chlorine are dissolved in 500 ml of dry THF, and added, dropwise, at 20° C, to a vigorously shaken solution of 0.45 moles of 1-(2-phenylethyl)-4-piperidinamethanamine in 500 ml of dry THF. After 2 hours at 20° C the white precipitate of chlorhydrate is separated. Yield: 85%. (MP: 221-5° C).

Example 10. N- [1-(2-PHENYLETHYL)-4-PIPERIDINYL] -2-THIOPHENEACETAMIDE

This product is obtained in the form of a chlorhydrate, a white crystalline solid, using the process described in Example 9. Yield: 90%. (MP: 207-10° C).

Example 11. N-[1-(1-METHYLETHYL)-4-PIPERIDINYL] -2-FURANECARBOXAMIDE

The reaction between 2-furanecarboxylic acid chloride and 1-(1-methylethyl)-4-piperidinamine in THF, in accordance with the conditions described in Example 9, gives the chlorhydrate of the corresponding carboxamide, which is extremely hygroscopic. A solution of the latter in $H_2O$ is treated with NaOH up to a pH of 8.5 to give a white crystalline solid which is the free base of the desired product. Yield: 80%. (MP: 150-2° C).

Example 12. N- [1-(2-PHENYLETHYL)-4-PIPERIDINYL] METHYL-3-THIOPHENECARBOXAMIDE

In accordance with the process described in Example 9, 3-thiophenecarboxylic acid chloride and 1-(2-phenylethyl)-4-piperidinemethanamine are reacted to give 90% of the carboxamide chlorhydrate, and amorphous and hygroscopic white solid. (MP: 225-8° C).

Example 13. 2-HYDROXY-N- [1-(2-PHENYLETHYL)-4-PIPERIDINYL] BENZENEACETAMIDE

The reaction between 2-hydroxy-benzeneacetic acid and 1-(2-phenylethyl)-4-piperidinamine in THF, in the presence of DCC, according to the process described in Example 1, leads to the obtention of this product, with a yield of 55%. The chlorhydrate is an extremely hygroscopic product and the free base is obtained by means of the standard technique. (MP: 163-5° C).

Example 14. 4-ACETYLOXY-N-[1-(2-PHENYLETHYL)-4-PIPERIDINYL]-BENZENEACETAMIDE

The title compound is obtained with Example 5, by reacting the chloride of 4-acetyloxy-benzeneacetic acid with 1-(2-phenylethyl)-4-piperidinamine in THF. The chlorhydrate is obtained with a yield of 75 - 80%. (MP: 100 - 201° C).

Example 15. 3-METHOXY-5-PHENYL-N-[1-(2-PHENYLETHYL)-4-PIPERIDINYL] METHYL-2-THIOPHENECARBOXAMIDE

From the chloride of 3-methoxy-5-phenyl-2-thiophenecarboxylic acid and 1-(2-phenylethyl)-4-piperidinamethanamine, and using the conditions described in Example 5, the corresponding chlorhydrate is

obtained with a yield of 90%. (MP: 216-19° C).

Example 16. N-(1-PHENYLMETHYL-4-PIPERIDINYL)-BENZENEPROPENAMIDE

By reacting a mixture of ethyl cynnamate and 1-phenylmethyl-4-piperidinamine in anhydrous xylene in the presence of a 1/10 M amount of 4-methylbenzenesulfonic acid at the reflux temperature (24 hours) in the proportions described in Example 4, the chlorhydrate of the corresponding carboxamide is obtained, with a yield of 60 - 65%. (MP:223-6° C).

Example 17. N-4-HYDROXYPHENYL-N′- [1-(2-PHENYLETHYL-4-PIPERIDINYL]-UREA

A solution of 0.11 moles of 4-hydroxyphenyl isocyanate in 150 ml of ethyl acetate is treated at 20° C, dropwise, with a solution of 1-(2-phenylethyl)-4-piperidinamine (0.11 mol) in 100 ml of ethyl acetate. The mixture is shaken at 20° C for 3 to 4 hours, and the resulting solid is filtered and washed with hexane, recrystallizing from EtOH-HCl. Chlorhydrate yield: 55%. (MP: 148 - 150° C).

Example 18. N-4-HYDROXYPHENYL-N′- [1-METHYLETHYL)-4-PIPERIDINYL]-UREA

Following an analogous process to that of Example 17, this product is obtained using 4-hydroxyphenyl isocyanate and 1-(1-methylethyl)-4-piperidinamine. Yield: 50%. (MP (HCl) : 260 - 2 (d)° C).

## Claims

1. - The compounds of general formula I, wherein: $R^1$ is preferably an alkylic ($C_1$ - $C_4$), alicyclic ($C_5$ - $C_6$), alkylarylic or alkylheteroarylic radical, as well as those resulting from the substitution in the aromatic ring with $CH_3$, $C_2H_5$, OH, $OCH_3$, $CF_3$, F, Cl, Br, I, $NH_2$ and $NO_2$, such as, for example: methyl, ethyl, propyl, isopropyl, cyclopentyl, cyclohexyl, 2-phenylethyl, 2-piperidinyl-methyl, 3-piperidinyl-methyl, 4-piperidinyl-methyl, 4-phenyl-4-hydroxy-butyl, 4-methoxyphenyl-4-hydroxy-butyl, 4-methoxy-phenylethyl, etc.;

| | |
|---|---|
| Z | can be Co, CONH; |
| n | can be 0, 1 or 2; |
| m | can be 0, 1 or 2; |
| $R^2$ | can be OH, H. |

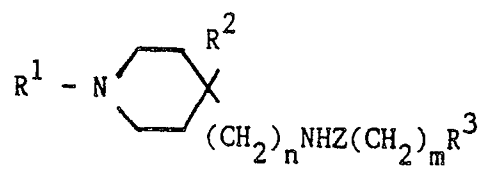

$R^3$ is preferably an arylic, alkylarylic, alkylheteroarylic or heteroarylic radical, as well as those resulting from the substitution in the aromatic ring with $CH_3$, $C_2H_5$, OH, $OCH_3$, $CF_3$, F, Cl, Br, I, $NH_2$, $NHCOCH_3$, $NHSO_2CH_3$, $N(CH_3)SO_2CH_3$, $NO_2$, COOH, $OCOCH_3$, $N(CH_3)_2$, etc., such as, for example, 4-chlorophenyl-methyl, 3-chlorophenyl-methyl, 2-chlorophenyl-methyl, 4-hydroxyphenyl, 4-hydroxyphenyl-methyl, 3-hydroxyphenyl-methyl, 2-hydroxyphenyl-methyl, 4-acetyloxyphenyl-methyl, 3-acetyloxyphenyl-methyl, 4-fluorophenyl-methyl, 3-fluorophenyl-methyl, 2-fluorophenyl-methyl, 4-methylphenyl-methyl, 3-methylphenyl-methyl, 2-methylphenyl-methyl, 2-thiophenemethyl, 3-thiophenemethyl, 2-thiophenemethyl, 2-(3-thiophenyl)-ethenyl, 2-furanemethyl, 3-furanemethyl, 2-pyridyl-methyl, 3-pyridyl-methyl, 4-pyridyl-methyl, 2-phenyl-ethenyl, 2-thiophenyl, 3-thiophenyl, 2-furanyl, 3-furanyl, 2-pyridyl, 3-pyridyl, 4-pyridyl, 2-thiazolyl, 5-thiazolyl, 2-(3-methyl)-thiophenyl, 2-(5-methyl)-thiophenyl, 2-(3-methoxy-5-methyl)-thiophenyl, 2-(3-methoxy-5-phenyl)-thiophenyl, 2-(3-methylsulfonylamino-methyl)-thiophenyl, 2-(3-methylsulfonylamine)-thiophenyl, 5-(2,4-dimethyl-thiazolyl), cyclohexyl, cyclopentyl, 1-cyclohexenyl, 1-cyclopentenyl, cyclohexyl-methyl, 1-(cyclohexenyl)-methyl, cyclopentyl-methyl, 1-(cyclopentenyl)-methyl, 2-tetrahydrothiophenyl, 2-tetrahydrofuranyl, 2-tetrahydrothiophenyl-methyl, 2-tetrahydrofuranyl-methyl, 4-dimethylaminophenyl-methyl, 4-aminophenyl-methyl, 2-aminophenyl-methyl, etc.

2.- A process for preparing the compounds of formula I, in accordance with Claim 1, wherein Z = CO, characterised in that a carboxylic acid of formula $R^3(CH_2)_mCOOH$ is reacted with a 4-piperidinalcanamine of formula II

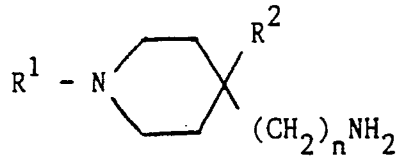

II

wherein $R^1$, $R^2$, $R^3$, m, n are as described in Claim 1, within a suitable solvent, such as dioxane, tetrahydrofurane, dimethylformamide or dimethylsulfoxide, in the presence of DCC.

3.- A process for preparing the compounds of formula I, in accordance with Claim 1, wherein Z = CO, characterised in that a 4-piperidinalcanamine of general formula II is reacted with an acid chloride of formula $R^3(CH_2)_mCOCl$, wherein $R^1$, $R^2$, $R^3$, m, n are as described in CLaim 1, in a suitable solvent such as ethyl ether, chloroform, dioxane, dichloromethane benzene or tetrahydrofurane.

4.- A process for preparing the compounds of formula I, in accordance with Claim 1, wherein Z = CO, characterised in that a 4-piperidinalcanamine of general formula II is reacted with an ester of formula $R^3$-$(CH_2)_mCOOR^6$, wherein $R^1$, $R^2$, $R^3$, m, n are as described in Claim 1, and $R^6$ is $CH_3$, $C_2H_5$ or $CH_3OCH_2CH_2$, in a solvent having a high boiling point, such as xylene, toluene or dimethylformamide.

5.- A process in accordance with the compounds of general formula I, in accordance with Claim 1, wherein Z = CONH, characterised in that an isocyanate of formula $R^3(CH_2)_mNCO$ is reacted with a 4-piperidinalcanamine of general formula II, wherein $R^1$, $R^2$, $R^3$, m, n are as described in Claim 1, in a suitable solvent, such as ethyl acetate, toluene, ethyl ether, or tetrahydrofurane.

6.- Compounds of formula 1, wherein:

$R^1$ = phenylmethyl, 2-phenylethyl, 1-methylethyl, methyl, ethyl, 4-hydroxy-4-phenylbutyl

$R^2$ = H, OH

n = 0

z = CO

in which the $R^3(CH_2)_m$ radical represents:

$C_6H_5CH=CH$-, $C_6H_5CH_2$-, 4-$CH_3COO$-$C_6H_4CH_2$, 3-$CH_3COO$-$C_6H_4CH_2$-, 2-$CH_3COO$-$C_6H_4$-$CH_2$-, 4-HO-$C_6H_4$-$CH_2$-, 3-HO-$C_6H_4$-$CH_2$-, 2-HO-$C_6H_4$-$CH_2$-, 3,4,5-$(CH_3O)$-$C_6H_2$-$CH_2CH_2$ 4-Cl-$C_6H_4$-$CH_2$-, 3-Cl-$C_6H_4CH_2$-, 2-Cl-$C_6H_4$-$CH_2$-, 4-F-$C_6H_4$-$CH_2$-, 3-F-$C_6H_4$-$CH_2$-, 2-F-$C_6H_4$-$CH_2$-, 4-HO-$C_6H_4CH_2CH_2$-, 4-$CH_3COO$-$C_6H_4CH_2CH_2$-4-$CH_3$-$C_6H_4$-$CH_2$-, 3-$CH_3$-$C_6H_4$-$CH_2$-, 2-$CH_3$-$C_6H_4$-$CH_2$-, 4-$CH_3O$-$C_6H_4$-$CH_2$-4-$CH_3O$-$C_6H_4$-$CH_2CH_2$-, 3-$CH_3O$-$C_6H_4CH_2$-, 2-$CH_3O$-$C_6H_4$-$CH_2$-, 2-thiophenyl-, 3-thiophenyl-, 2-(2-thiophenyl)-ethene-1-yl, 2-(3-thiophenyl)-ethene-1-yl-, 2-thiophenemethyl-, 3-thiophenemethyl-3-methyl-2-thiophenyl-, 5-methyl-2-thiophenyl-, 3-hydroxy-5-methyl-2-thiophenyl-, 3-hydroxy-5-phenyl-2-thiophenyl-, 3-methoxy-5-methyl-2-thiophenyl-, 3-methoxy-5-phenyl-2-thiophenyl, 3-hydroxy-2-thiophenyl-, 3-methylsulfonylamino-2-thiophenyl-, 3-methylsulfonylaminomethyl-2-thiophenyl-, 3-dimethylamino-2-thiophenyl-, 2,4-dimethyl-5-thiazolyl-, 2-tetrahydrothiophenyl-, 3-tetrahydrothiophenyl-, 2-tetrahydrothiophenylmethyl-, 3-tetrahydrothiophenylmethyl-, 2-furanyl-, 3-furanyl-, 2-furanylmethyl-, 3-furanylmethyl-, 2-tetrahydrofuranyl-, 3-tetrahydrofuranyl-, 2-pyridyl, 3-pyridyl, 4-pyridyl, 2-pyridylmethyl-, 3-pyridylmethyl-, 4-pyridylmethyl-, cyclohexyl, cyclopentyl, 1-cyclohexenyl-, 1-cyclopentenyl-, 1-cyclohexenylmethyl-1-cyclopentenylmethyl-, cyclohexylmethyl-, cyclopentylmethyl-, 4-$H_2NC_6H_4CH_2$-, 3-$H_2NC_6H_4CH_2$-, 2-$H_2N$-$C_6H_4CH_2$-, 4-$(CH_3)_2N$-$C_6H_4$-CH2-3-$(CH_3)_2N$-$C_6H_4$-$CH_2$-, 2$(CH_3)_2N$-$C_6H_4$-$CH_2$-, 4-$CH_3CONH$-$C_6H_4CH_2$-, 2-$CH_3CONHC_6H_4$-$CH_2$-, 4-$CH_3SO_2NH$-$C_6H_4CH_2$-.

7.- Compounds of formula I, wherein:

$R^1$ = phenylmethyl, 2-phenylethyl, 1-methylethyl, methyl, ethyl, 4-hydroxy-4-phenylbutyl

$R^2$ = H, OH

n = 1

z = CO

in which the $R^3(CH_2)_m$ radical represents:

$C_6H_5CH=CH$-, $C_6H_5CH_2$-, 4-$CH_3COO$-$C_6H_4CH_2$, 3-$CH_3COO$-$C_6H_4CH_2$-, 2-$CH_3COO$-$C_6H_4$-$CH_2$-,4-HO-$C_6H_4$-$CH_2$-, 3-HO-$C_6H_4$-$CH_2$-, 2-HO-$C_6H_4$-$CH_2$-, 3,4,5-$(CH_3O)$-$C_6H_2$-$CH_2CH_2$ 4-Cl-$C_6H_4$-$CH_2$-, 3-Cl-$C_6H_4CH_2$-, 2-Cl-$C_6H_4$-$CH_2$-, 4-F-$C_6H_4$-$CH_2$-, 3-F-$C_6H_4$-$CH_2$-, 2-F-$C_6H_4$-$CH_2$-, 4-HO-$C_6H_4CH_2CH_2$-, 4-$CH_3COO$-$C_6H_4CH_2CH_2$-4-$CH_3$-$C_6H_4$-$CH_2$-, 3-$CH_3$-$C_6H_4$-$CH_2$-, 2-$CH_3$-$C_6H_4$-$CH_2$-, 4-$CH_3O$-$C_6H_4$-$CH_2$-4-$CH_3O$-$C_6H_4$-$CH_2CH_2$-, 3-$CH_3O$-$C_6H_4CH_2$-, 2-$CH_3O$-$C_6H_4$-$CH_2$-, 2-thiophenyl-, 3-thiophenyl-, 2-(2-thiophenyl)-ethene-1-yl,

2-(3-thiophenyl)-ethene-1-yl-, 2-thiophenemethyl-, 3-thiophenemethyl-3-methyl-2-thiophenyl-, 5-methyl-2-thiophenyl-, 3-hydroxy-5-methyl-2-thiophenyl-, 3-hydroxy-5-phenyl-2-thiophenyl-, 3-methoxy-5-methyl-2-thiophenyl-, 3-methoxy-5-phenyl-2-thiophenyl, 3-hydroxy-2-thiophenyl-, 3-methylsulfonylamino-2-thiophenyl-, 3-methylsulfonylaminomethyl-2-thiophenyl-, 3-dimethylamino-2-thiophenyl-, 2,4-dimethyl-5-thiazolyl-, 2-tetrahydrothiophenyl-, 3-tetrahydrothiophenyl-, 2-tetrahydrothiophenylmethyl-, 3-tetrahydrothiophenylmethyl-, 2-furanyl-, 3-furanyl-, 2-furanylmethyl-, 3-furanylmethyl-, 2-tetrahydrofuranyl-, 3-tetrahydrofuranyl-, 2-pyridyl, 3-pyridyl, 4-pyridyl, 2-pyridylmethyl-, 3-pyridylmethyl-, 4-pyridylmethyl-, cyclohexyl, cyclopentyl, 1-cyclohexenyl-, 1-cyclopentenyl-, 1-cyclohexenylmethyl-1-cyclopentenylmethyl-, cyclohexylmethyl-, cyclopentylmethyl-, 4-$H_2NC_6H_4CH_2$-, 3-$H_2NC_6H_4CH_2$-, 2-$H_2N$-$C_6H_4CH_2$-, 4-$(CH_3)_2N$-$C_6H_4$-CH2-3-$(CH_3)_2N$-$C_6H_4$-$CH_2$-, 2$(CH_3)_2N$-$C_6H_4$-$CH_2$-, 4-$CH_3CONH$-$C_6H_4CH_2$-, 2-$CH_3CONHC_6H_4$-$CH_2$-, 4-$CH_3SO_2NH$-$C_6H_4CH_2$-.

8.- Compounds of formula I, wherein:

$R^1$ = phenylmethyl, 2-phenylethyl, 1-methylethyl, methyl, ethyl, 4-hydroxy-4-phenylbutyl

$R^2$ = H, OH

n = 2

z = CO

in which the $R^3(CH_2)_m$ radical represents:

$C_6H_5CH=CH$-, $C_6H_5CH_2$-, 4-$CH_3COO$-$C_6H_4CH_2$, 3-$CH_3COO$-$C_6H_4CH_2$-, 2-$CH_3COO$-$C_6H_4$-$CH_2$-, 4-HO-$C_6H_4$-$CH_2$-, 3-HO-$C_6H_4$-$CH_2$-, 2-HO-$C_6H_4$-$CH_2$-, 3,4,5-$(CH_3O)$-$C_6H_2$-$CH_2CH_2$ 4-Cl-$C_6H_4$-$CH_2$-, 3-Cl-$C_6H_4CH_2$-, 2-Cl-$C_6H_4$-$CH_2$-, 4-F-$C_6H_4$-$CH_2$-, 3-F-$C_6H_4$-$CH_2$-, 2-F-$C_6H_4$-$CH_2$-, 4-HO-$C_6H_4CH_2CH_2$-, 4-$CH_3COO$-$C_6H_4CH_2CH_2$-4-$CH_3$-$C_6H_4$-$CH_2$-, 3-$CH_3$-$C_6H_4$-$CH_2$-, 2-$CH_3$-$C_6H_4$-$CH_2$-, 4-$CH_3O$-$C_6H_4$-$CH_2$-4-$CH_3O$-$C_6H_4$-$CH_2CH_2$-, 3-$CH_3O$-$C_6H_4CH_2$-, 2-$CH_3O$-$C_6H_4$-$CH_2$-, 2-thiophenyl-, 3-thiophenyl-, 2-(2-thiophenyl)-ethene-1-yl, 2-(3-thiophenyl)-ethene-1-yl-, 2-thiophenemethyl-, 3-thiophenemethyl-3-methyl-2-thiophenyl-, 5-methyl-2-thiophenyl-, 3-hydroxy-5-methyl-2-thiophenyl-, 3-hydroxy-5-phenyl-2-thiophenyl-, 3-methoxy-5-methyl-2-thiophenyl-, 3-methoxy-5-phenyl-2-thiophenyl, 3-hydroxy-2-thiophenyl-, 3-methylsulfonylamino-2-thiophenyl-, 3-methylsulfonylaminomethyl-2-thiophenyl-, 3-dimethylamino-2-thiophenyl-, 2,4-dimethyl-5-thiazolyl-, 2-tetrahydrothiophenyl-, 3-tetrahydrothiophenyl-, 2-tetrahydrothiophenylmethyl-, 3-tetrahydrothiophenylmethyl-, 2-furanyl-, 3-furanyl-, 2-furanylmethyl-, 3-furanylmethyl-, 2-tetrahydrofuranyl-, 3-tetrahydrofuranyl-, 2-pyridyl, 3-pyridyl, 4-pyridyl, 2-pyridylmethyl-, 3-pyridylmethyl-, 4-pyridylmethyl-, cyclohexyl, cyclopentyl, 1-cyclohexenyl-, 1-cyclopentenyl-, 1-cyclohexenylmethyl-1-cyclopentenylmethyl-, cyclohexylmethyl-, cyclopentylmethyl-, 4-$H_2NC_6H_4CH_2$-, 3-$H_2NC_6H_4CH_2$-, 2-$H_2N$-$C_6H_4CH_2$ -,4-$(CH_3)_2N$-$C_6H_4$-CH2-3-$(CH_3)_2N$-$C_6H_4$-$CH_2$-, 2$(CH_3)_2N$-$C_6H_4$-$CH_2$-, 4-$CH_3CONH$-$C_6H_4CH_2$-, 2-$CH_3CONHC_6H_4$-$CH_2$-, 4-$CH_3SO_2NH$-$C_6H_4CH_2$-.

9.- Compounds of formula I, wherein:

$R^1$ = phenylmethyl, 2-phenylethyl, 1-methylethyl, methyl, ethyl, 4-hydroxy-4-phenylbutyl

$R^2$ = H, OH

n = 0

z = CONH

in which the $R^3(CH_2)_m$ radical represents:

$C_6H_5CH=CH$-, $C_6H_5CH_2$-, 4-$CH_3COO$-$C_6H_4CH_2$, 3-$CH_3COO$-$C_6H_4CH_2$-, 2-$CH_3COO$-$C_6H_4$-$CH_2$-, 4-HO-$C_6H_4$-$CH_2$-, 3-HO-$C_6H_4$-$CH_2$-, 2-HO-$C_6H_4$-$CH_2$-, 3,4,5-$(CH_3O)$-$C_6H_2$-$CH_2CH_2$ 4-Cl-$C_6H_4$-$CH_2$-, 3-Cl-$C_6H_4CH_2$-, 2-Cl-$C_6H_4$-$CH_2$-, 4-F-$C_6H_4$-$CH_2$-, 3-F-$C_6H_4$-$CH_2$-, 2-F-$C_6H_4$-$CH_2$-, 4-HO-$C_6H_4CH_2CH_2$-, 4-$CH_3COO$-$C_6H_4CH_2CH_2$-4-$CH_3$-$C_6H_4$-$CH_2$-, 3-$CH_3$-$C_6H_4$-$CH_2$-, 2-$CH_3$-$C_6H_4$-$CH_2$-, 4-$CH_3O$-$C_6H_4$-$CH_2$-4-$CH_3O$-$C_6H_4$-$CH_2CH_2$-, 3-$CH_3O$-$C_6H_4CH_2$-, 2-$CH_3O$-$C_6H_4$-$CH_2$-, 2-thiophenyl-, 3-thiophenyl-, 2-(2-thiophenyl)-ethene-1-yl, 2-(3-thiophenyl)-ethene-1-yl-, 2-thiophenemethyl-, 3-thiophenemethyl-3-methyl-2-thiophenyl-, 5-methyl-2-thiophenyl-, 3-hydroxy-5-methyl-2-thiophenyl-, 3-hydroxy-5-phenyl-2-thiophenyl-, 3-methoxy-5-methyl-2-thiophenyl-, 3-methoxy-5-phenyl-2-thiophenyl, 3-hydroxy-2-thiophenyl-, 3-methylsulfonylamino-2-thiophenyl-, 3-methylsulfonylaminomethyl-2-thiophenyl-, 3-dimethylamino-2-thiophenyl-, 2,4-dimethyl-5-thiazolyl-, 2-tetrahydrothiophenyl-, 3-tetrahydrothiophenyl-, 2-tetrahydrothiophenylmethyl-, 3-tetrahydrothiophenylmethyl-, 2-furanyl-, 3-furanyl-, 2-furanylmethyl-, 3-furanylmethyl-, 2-tetrahydrofuranyl-, 3-tetrahydrofuranyl-, 2-pyridyl, 3-pyridyl, 4-pyridyl, 2-pyridylmethyl-, 3-pyridylmethyl-, 4-pyridylmethyl-, cyclohexyl, cyclopentyl, 1-cyclohexenyl-, 1-cyclopentenyl-, 1-cyclohexenylmethyl-1-cyclopentenylmethyl-, cyclohexylmethyl-, cyclopentylmethyl-, 4-$H_2NC_6H_4CH_2$-, 3-$H_2NC_6H_4CH_2$-, 2-$H_2N$-$C_6H_4CH_2$-, 4-$(CH_3)_2N$-$C_6H_4$-CH2-3-$(CH_3)_2N$-$C_6H_4$-$CH_2$-, 2$(CH_3)_2N$-$C_6H_4$-$CH_2$-, 4-$CH_3CONH$-$C_6H_4CH_2$-, 2-$CH_3CONHC_6H_4$-$CH_2$-, 4-$CH_3SO_2NH$-$C_6H_4CH_2$-.

10.- Compounds of formula I, wherein:

$R^1$ = phenylmethyl, 2-phenylethyl, 1-methylethyl, methyl, ethyl, 4-hydroxy-4-phenylbutyl

$R^2$ = H, OH

n = 1
z = CONH
in which the $R^3(CH_2)_m$ radical represents:
$C_6H_5CH=CH$-, $C_6H_5CH_2$-, 4-$CH_3COO$-$C_6H_4CH_2$, 3-$CH_3COO$-$C_6H_4CH_2$-, 2-$CH_3COO$-$C_6H_4$-$CH_2$-, 4-HO-$C_6H_4$-$CH_2$-, 3-HO-$C_6H_4$-$CH_2$-, 2-HO-$C_6H_4$-$CH_2$-, 3,4,5-($CH_3O$)-$C_6H_2$-$CH_2CH_2$ 4-Cl-$C_6H_4$-$CH_2$-, 3-Cl-$C_6H_4CH_2$-, 2-Cl-$C_6H_4$-$CH_2$-, 4-F-$C_6H_4$-$CH_2$-, 3-F-$C_6H_4$-$CH_2$-, 2-F-$C_6H_4$-$CH_2$-, 4-HO-$C_6H_4CH_2CH_2$-, 4-$CH_3C$ OO-$C_6H_4CH_2CH_2$-4-$CH_3$-$C_6H_4$-$CH_2$-, 3-$CH_3$-$C_6H_4$-$CH_2$-, 2-$CH_3$-$C_6H_4$-$CH_2$-, 4-$CH_3O$-$C_6H_4$-$CH_2$-4-$CH_3O$-$C_6H_4$-$CH_2CH_2$-, 3-$CH_3O$-$C_6H_4CH_2$-, 2-$CH_3O$-$C_6H_4$-$CH_2$-, 2-thiophenyl-, 3-thiophenyl-, 2-(2-thiophenyl)-ethene-1-yl, 2-(3-thiophenyl)-ethene-1-yl-, 2-thiophenemethyl-, 3-thiophenemethyl-3-methyl-2-thiophenyl-, 5-methyl-2-thiophenyl-, 3-hydroxy-5-methyl-2-thiophenyl-, 3-hydroxy-5-phenyl-2-thiophenyl-, 3-methoxy-5-methyl-2-thiophenyl-, 3-methoxy-5-phenyl-2-thiophenyl, 3-hydroxy-2-thiophenyl-, 3-methylsulfonylamino-2-thiophenyl-, 3-methylsulfonylaminomethyl-2-thiophenyl-, 3-dimethylamino-2-thiophenyl-, 2,4-dimethyl-5-thiazolyl-, 2-tetrahydrothiophenyl-, 3-tetrahydrothiophenyl-, 2-tetrahydrothiophenylmethyl-, 3-tetrahydrothiophenylmethyl-, 2-furanyl-, 3-furanyl-, 2-furanylmethyl-, 3-furanylmethyl-, 2-tetrahydrofuranyl-, 3-tetrahydrofuranyl-, 2-pyridyl, 3-pyridyl, 4-pyridyl, 2-pyridylmethyl-, 3-pyridylmethyl-, 4-pyridylmethyl-, cyclohexyl, cyclopentyl, 1-cyclohexenyl-, 1-cyclopentenyl-, 1-cyclohexenylmethyl-1-cyclopentenylmethyl-, cyclohexylmethyl-, cyclopentylmethyl-, 4-$H_2NC_6H_4CH_2$-, 3-$H_2NC_6H_4CH_2$-, 2-$H_2N$-$C_6H_4CH_2$-, 4-$(CH_3)_2N$-$C_6H_4$-CH2-3-$(CH_3)_2N$-$C_6H_4$-$CH_2$-, 2$(CH_3)_2N$-$C_6H_4$-$CH_2$-, 4-$CH_3CONH$-$C_6H_4CH_2$-, 2-$CH_3CONHC_6H_4$-$CH_2$-, 4-$CH_3SO_2NH$-$C_6H_4CH_2$-.

11.- Compounds of formula I, wherein:
$R^1$ = phenylmethyl, 2-phenylethyl, 1-methylethyl, methyl, ethyl, 4-hydroxy-4-phenylbutyl
$R^2$ = H, OH
n = 2
z = CONH
in which the $R^3(CH_2)_m$ radical represents:
$C_6H_5CH=CH$-, $C_6H_5CH_2$-, 4-$CH_3COO$-$C_6H_4CH_2$, 3-$CH_3COO$-$C_6H_4CH_2$-, 2-$CH_3COO$-$C_6H_4$-$CH_2$-, 4-HO-$C_6H_4$-$CH_2$-, 3-HO-$C_6H_4$-$CH_2$-, 2-HO-$C_6H_4$-$CH_2$-, 3,4,5-($CH_3O$)-$C_6H_2$-$CH_2CH_2$ 4-Cl-$C_6H_4$-$CH_2$-, 3-Cl-$C_6H_4CH_2$-, 2-Cl-$C_6H_4$-$CH_2$-, 4-F-$C_6H_4$-$CH_2$-, 3-F-$C_6H_4$-$CH_2$-, 2-F-$C_6H_4$-$CH_2$-, 4-HO-$C_6H_4CH_2CH_2$-, 4-$CH_3C$ OO-$C_6H_4CH_2CH_2$-4-$CH_3$-$C_6H_4$-$CH_2$-, 3-$CH_3$-$C_6H_4$-$CH_2$-, 2-$CH_3$-$C_6H_4$-$CH_2$-, 4-$CH_3O$-$C_6H_4$-$CH_2$-4-$CH_3O$-$C_6H_4$-$CH_2CH_2$-, 3-$CH_3O$-$C_6H_4CH_2$-, 2-$CH_3O$-$C_6H_4$-$CH_2$-, 2-thiophenyl-, 3-thiophenyl-, 2-(2-thiophenyl)-ethene-1-yl, 2-(3-thiophenyl)-ethene-1-yl-, 2-thiophenemethyl-, 3-thiophenemethyl-3-methyl-2-thiophenyl-, 5-methyl-2-thiophenyl-, 3-hydroxy-5-methyl-2-thiophenyl-, 3-hydroxy-5-phenyl-2-thiophenyl-, 3-methoxy-5-methyl-2-thiophenyl-, 3-methoxy-5-phenyl-2-thiophenyl, 3-hydroxy-2-thiophenyl-, 3-methylsulfonylamino-2-thiophenyl-, 3-methylsulfonylaminomethyl-2-thiophenyl-, 3-dimethylamino-2-thiophenyl-, 2,4-dimethyl-5-thiazolyl-, 2-tetrahydrothiophenyl-, 3-tetrahydrothiophenyl-, 2-tetrahydrothiophenylmethyl-, 3-tetrahydrothiophenylmethyl-, 2-furanyl-, 3-furanyl-, 2-furanylmethyl-, 3-furanylmethyl-, 2-tetrahydrofuranyl-, 3-tetrahydrofuranyl-, 2-pyridyl, 3-pyridyl, 4-pyridyl, 2-pyridylmethyl-, 3-pyridylmethyl-, 4-pyridylmethyl-, cyclohexyl, cyclopentyl, 1-cyclohexenyl-, 1-cyclopentenyl-, 1-cyclohexenylmethyl-1-cyclopentenylmethyl-, cyclohexylmethyl-, cyclopentylmethyl-, 4-$H_2NC_6H_4CH_2$-, 3-$H_2NC_6H_4CH_2$-, 2-$H_2N$-$C_6H_4CH_2$-, 4-$(CH_3)_2N$-$C_6H_4$-CH2-3-$(CH_3)_2N$-$C_6H_4$-$CH_2$-, 2$(CH_3)_2N$-$C_6H_4$-$CH_2$-, 4-$CH_3CONH$-$C_6H_4CH_2$-, 2-$CH_3CONHC_6H_4$-$CH_2$-, 4-$CH_3SO_2NH$-$C_6H_4CH_2$-.

12.- Compounds of formula I, according to Claims 6, 7, 8, 9, 10 and 11, as well as pharmaceutically acceptable salts thereof, preferably chlohydrate, bromhydrate, citrate, tartrate and maleate.

Amended claims in accordance with Rule 86(2) EPC.

1. - The compounds of general formula I, wherein:
$R^1$ is preferably a lowe alkyl radical containing up to 3 carbon atoms such as methyl, ethyl and 1-4methylethyl. An aryl-alkylene grouping AR-$CH_2$-$CH_2$ in wich AR may have tge following meanings; phenyl or phenyl radical substituted by from 1 to 2 of the following substituents; F, CL, Br, I, $CH_3$, $C_2Hs$, OH, $Och_3$, $CF_3$, $NH_2$ and $NO_2$.

Z can be Co, CONH;
n can be 0, 1 or 2;
m can be 0, 1 or 2;
$R^2$ can be OH, H.

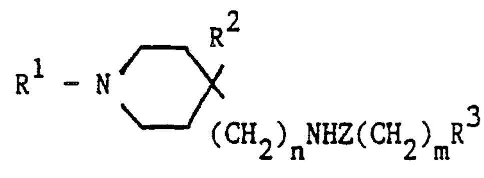

$R^3$ is preferably an alkylarylic, alkylheteroarylic or heteroarylic radical, as well as those resulting from the substitution in the aromatic ring with $CH_3$, $C_2H_5$, OH, $OCH_3$, $CF_3$, F, Cl, Br, I, $NH_2$, $NHCOCH_3$, $NHSO_2CH_3$, $N(CH_3)SO_2CH_3$, $NO_2$, COOH, $OCOCH_3$, $N(CH_3)_2$, etc., such as, for example, 4-chlorophenyl-methyl, 3-chlorophenyl-methyl, 2-chlorophenyl-methyl, 4-hydroxyphenyl, 4-hydroxyphenyl-methyl, 3-hydroxyphenyl-methyl, 2-hydroxyphenyl-methyl, 4-acetyloxyphenyl-methyl, 3-acetyloxyphenyl-methyl, 4-fluorophenyl-methyl, 3-fluorophenyl-methyl, 2-fluorophenyl-methyl, 4-methylphenyl-methyl, 3-methylphenyl-methyl, 2-methylphenyl-methyl, 2-thiophenemethyl, 3-thiophenemethyl, 2-thiophenemethyl, 2-(3-thiophenyl)-ethenyl, 2-furanemethyl, 3-furanemethyl, 2-pyridyl-methyl, 3-pyridyl-methyl, 4-pyridyl-methyl, 2-phenyl-ethenyl, 2-thiophenyl, 3-thiophenyl, 2-furanyl, 3-furanyl, 2-pyridyl, 3-pyridyl, 4-pyridyl, 2-thiazolyl, 5-thiazolyl, 2-(3-methyl)-thiophenyl, 2-(5-methyl)-thiophenyl, 2-(3-methoxy-5-methyl)-thiophenyl, 2-(3-methoxy-5-phenyl)-thiophenyl, 2-(3-methylsulfonylamino-methyl)-thiophenyl, 2-(3-methylsulfonylamine)-thiophenyl, 5-(2,4-dimethyl-thiazolyl), cyclohexyl, cyclopentyl, 1-cyclohexenyl, 1-cyclopentenyl, cyclohexyl-methyl, 1-(cyclohexenyl)-methyl, cyclopentyl-methyl, 1-(cyclopentenyl)-methyl, 2-tetrahydrothiophenyl, 2-tetrahydrofuranyl, 2-tetrahydrothiophenyl-methyl, 2-tetrahydrofuranyl-methyl, 4-dimethylaminophenyl-methyl, 4-aminophenyl-methyl, 2-aminophenyl-methyl, etc.

2.- A process for preparing the compounds of formula I, in accordance with Claim 1, wherein Z = CO, characterised in that a carboxylic acid of formula $R^3(CH_2)_mCOOH$ is reacted with a 4-piperidinalcanamine of formula II

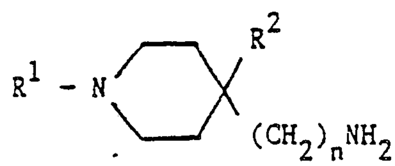

II

wherein $R^1$, $R^2$, $R^3$, m, n are as described in Claim 1, within a suitable solvent, such as dioxane, tetrahydrofurane, dimethylformamide or dimethylsulfoxide, in the presence of DCC.

3.- A process for preparing the compounds of formula I, in accordance with Claim 1, wherein Z = CO, characterised in that a 4-piperidinalcanamine of general formula II is reacted with an acid chloride of formula $R^3(CH_2)_mCOCl$, wherein $R^1$, $R^2$, $R^3$, m, n are as described in CLaim 1, in a suitable solvent such as ethyl ether, chloroform, dioxane, dichloromethane benzene or tetrahydrofurane.

4.- A process for preparing the compounds of formula I, in accordance with Claim 1, wherein Z = CO, characterised in that a 4-piperidinalcanamine of general formula II is reacted with an ester of formula $R^3$-$(CH_2)_mCOOR^6$, wherein $R^1$, $R^2$, $R^3$, m, n are as described in Claim 1, and $R^6$ is $CH_3$, $C_2H_5$ or $CH_3OCH_2CH_2$, in a solvent having a high boiling point, such as xylene, toluene or dimethylformamide.

5.- A process in accordance with the compounds of general formula I, in accordance with Claim 1, wherein Z = CONH, characterised in that an isocyanate of formula $R^3(CH_2)_mNCO$ is reacted with a 4-piperidinalcanamine of general formula II, wherein $R^1$, $R^2$, $R^3$, m, n are as described in Claim 1, in a suitable solvent, such as ethyl acetate, toluene, ethyl ether, or tetrahydrofurane.

6.- Compounds of formula I, wherein:
$R^1$ = phenylmethyl, 2-phenylethyl, 1-methylethyl, methyl, ethyl,
$R^2$ = H, OH
n = 0
z = CO
in which the $R^3(CH_2)_m$ radical represents:
$C_6H_5CH=CH$-, $C_6H_5CH_2$-, 4-$CH_3COO$-$C_6H_4CH_2$, 3-$CH_3COO$-$C_6H_4CH_2$-, 2-$CH_3COO$-$C_6H_4$-$CH_2$-, 4-HO-$C_6H_4$-$CH_2$-, 3-HO-$C_6H_4$-$CH_2$-, 2-HO-$C_6H_4$-$CH_2$-, 3,4,5-$(CH_3O)$-$C_6H_2$-$CH_2CH_2$ 4-Cl-$C_6H_4$-$CH_2$-, 3-Cl-$C_6H_4CH_2$-, 2-Cl-$C_6H_4$-$CH_2$-, 4-F-$C_6H_4$-$CH_2$-, 3-F-$C_6H_4$-$CH_2$-, 2-F-$C_6H_4$-$CH_2$-, 4-HO-$C_6H_4CH_2CH_2$-, 4-$CH_3COO$-$C_6H_4CH_2CH_2$-4-$CH_3$-$C_6H_4$-$CH_2$-, 3-$CH_3$-$C_6H_4$-$CH_2$-, 2-$CH_3$-$C_6H_4$-$CH_2$-, 4-$CH_3O$-$C_6H_4$-$CH_2$-4-$CH_3O$-

$C_6H_4$-$CH_2CH_2$-, 3-$CH_3O$-$C_6H_4CH_2$-, 2-$CH_3O$-$C_6H_4$-$CH_2$-, 2-thiophenyl-, 3-thiophenyl-, 2-(2-thiophenyl)-ethene-1-yl, 2-(3-thiophenyl)-ethene-1-yl-, 2-thiophenemethyl-, 3-thiophenemethyl-3-methyl-2-thiophenyl-, 5-methyl-2-thiophenyl-, 3-hydroxy-5-methyl-2-thiophenyl-, 3-hydroxy-5-phenyl-2-thiophenyl-, 3-methoxy-5-methyl-2-thiophenyl-, 3-methoxy-5-phenyl-2-thiophenyl, 3-hydroxy-2-thiophenyl-, 3-methylsulfonylamino-2-thiophenyl-, 3-methylsulfonylaminomethyl-2-thiophenyl-, 3-dimethylamino-2-thiophenyl-, 2,4-dimethyl-5-thiazolyl-, 2-tetrahydrothiophenyl-, 3-tetrahydrothiophenyl-, 2-tetrahydrothiophenylmethyl-, 3-tetrahydrothiophenylmethyl-, 2-furanyl-, 3-furanyl-, 2-furanylmethyl-, 3-furanylmethyl-, 2-tetrahydrofuranyl-, 3-tetrahydrofuranyl-, 2-pyridyl, 3-pyridyl, 4-pyridyl, 2-pyridylmethyl-, 3-pyridylmethyl-, 4-pyridylmethyl-, cyclohexyl, cyclopentyl, 1-cyclohexenyl-, 1-cyclopentenyl-, 1-cyclohexenylmethyl-1-cyclopentenylmethyl-, cyclohexylmethyl-, cyclopentylmethyl-, 4-$H_2NC_6H_4CH_2$-, 3-$H_2NC_6H_4CH_2$-, 2-$H_2N$-$C_6H_4CH_2$-, 4-$(CH_3)_2N$-$C_6H_4$-CH2-3-$(CH_3)_2N$-$C_6H_4$-$CH_2$-, 2$(CH_3)_2N$-$C_6H_4$-$CH_2$-, 4-$CH_3CONH$-$C_6H_4CH_2$-, 2-$CH_3CONHC_6H_4$-$CH_2$-, 4-$CH_3SO_2NH$-$C_6H_4CH_2$-.

7.- Compounds of formula I, wherein:

$R^1$ = phenylmethyl, 2-phenylethyl, 1-methylethyl, methyl, ethyl,

$R^2$ = H, OH

n = 1

z = CO

in which the $R^3(CH_2)_m$ radical represents:

$C_6H_5CH=CH$-, $C_6H_5CH_2$-, 4-$CH_3COO$-$C_6H_4CH_2$, 3-$CH_3COO$-$C_6H_4CH_2$-, 2-$CH_3COO$-$C_6H_4$-$CH_2$-,4-HO-$C_6H_4$-$CH_2$-, 3-HO-$C_6H_4$-$CH_2$-, 2-HO-$C_6H_4$-$CH_2$-, 3,4,5-$(CH_3O)$-$C_6H_2$-$CH_2CH_2$ 4-Cl-$C_6H_4$-$CH_2$-, 3-Cl-$C_6H_4CH_2$-, 2-Cl-$C_6H_4$-$CH_2$-, 4-F-$C_6H_4$-$CH_2$-, 3-F-$C_6H_4$-$CH_2$-, 2-F-$C_6H_4$-$CH_2$-, 4-HO-$C_6H_4CH_2CH_2$-, 4-$CH_3COO$-$C_6H_4CH_2CH_2$-4-$CH_3$-$C_6H_4$-$CH_2$-, 3-$CH_3$-$C_6H_4$-$CH_2$-, 2-$CH_3$-$C_6H_4$-$CH_2$-, 4-$CH_3O$-$C_6H_4$-$CH_2$-4-$CH_3O$-$C_6H_4$-$CH_2CH_2$-, 3-$CH_3O$-$C_6H_4CH_2$-, 2-$CH_3O$-$C_6H_4$-$CH_2$-, 2-thiophenyl-, 3-thiophenyl-, 2-(2-thiophenyl)-ethene-1-yl, 2-(3-thiophenyl)-ethene-1-yl-, 2-thiophenemethyl-, 3-thiophenemethyl-3-methyl-2-thiophenyl-, 5-methyl-2-thiophenyl-, 3-hydroxy-5-methyl-2-thiophenyl-, 3-hydroxy-5-phenyl-2-thiophenyl-, 3-methoxy-5-methyl-2-thiophenyl-, 3-methoxy-5-phenyl-2-thiophenyl, 3-hydroxy-2-thiophenyl-, 3-methylsulfonylamino-2-thiophenyl-, 3-methylsulfonylaminomethyl-2-thiophenyl-, 3-dimethylamino-2-thiophenyl-, 2,4-dimethyl-5-thiazolyl-, 2-tetrahydrothiophenyl-, 3-tetrahydrothiophenyl-, 2-tetrahydrothiophenylmethyl-, 3-tetrahydrothiophenylmethyl-, 2-furanyl-, 3-furanyl-, 2-furanylmethyl-, 3-furanylmethyl-, 2-tetrahydrofuranyl-, 3-tetrahydrofuranyl-, 2-pyridylmethyl-, 3-pyridylmethyl-, 4-pyridylmethyl-, cyclopentyl, 1-cyclohexenyl-, 1-cyclopentenyl-, 1-cyclohexenylmethyl-1-cyclopentenylmethyl-, cyclopentylmethyl-, 4-$H_2NC_6H_4CH_2$-, 3-$H_2NC_6H_4CH_2$-, 2-$H_2N$-$C_6H_4CH_2$-, 4-$(CH_3)_2N$-$C_6H_4$-CH2-3-$(CH_3)_2N$-$C_6H_4$-$CH_2$-, 2$(CH_3)_2N$-$C_6H_4$-$CH_2$-, 4-$CH_3CONH$-$C_6H_4CH_2$-, 2-$CH_3CONHC_6H_4$-$CH_2$-, 4-$CH_3SO_2NH$-$C_6H_4CH_2$-.

8.- Compounds of formula I, wherein:

$R^1$ = phenylmethyl, 2-phenylethyl, 1-methylethyl, methyl, ethyl, $R^2$ = H, OH

n = 2

z = CO

in which the $R^3(CH_2)_m$ radical represents:

$C_6H_5CH=CH$-, $C_6H_5CH_2$-, 4-$CH_3COO$-$C_6H_4CH_2$, 3-$CH_3COO$-$C_6H_4CH_2$-, 2-$CH_3COO$-$C_6H_4$-$CH_2$-, 4-HO-$C_6H_4$-$CH_2$-, 3-HO-$C_6H_4$-$CH_2$-, 2-HO-$C_6H_4$-$CH_2$-, 3,4,5-$(CH_3O)$-$C_6H_2$-$CH_2CH_2$ 4-Cl-$C_6H_4$-$CH_2$-, 3-Cl-$C_6H_4CH_2$-, 2-Cl-$C_6H_4$-$CH_2$-, 4-F-$C_6H_4$-$CH_2$-, 3-F-$C_6H_4$-$CH_2$-, 2-F-$C_6H_4$-$CH_2$-, 4-HO-$C_6H_4CH_2CH_2$-, 4-$CH_3COO$-$C_6H_4CH_2CH_2$-4-$CH_3$-$C_6H_4$-$CH_2$-, 3-$CH_3$-$C_6H_4$-$CH_2$-, 2-$CH_3$-$C_6H_4$-$CH_2$-, 4-$CH_3O$-$C_6H_4$-$CH_2$-4-$CH_3O$-$C_6H_4$-$CH_2CH_2$-, 3-$CH_3O$-$C_6H_4CH_2$-, 2-$CH_3O$-$C_6H_4$-$CH_2$-, 2-thiophenyl-, 3-thiophenyl-, 2-(2-thiophenyl)-ethene-1-yl, 2-(3-thiophenyl)-ethene-1-yl-, 2-thiophenemethyl-, 3-thiophenemethyl-3-methyl-2-thiophenyl-, 5-methyl-2-thiophenyl-, 3-hydroxy-5-methyl-2-thiophenyl-, 3-hydroxy-5-phenyl-2-thiophenyl-, 3-methoxy-5-methyl-2-thiophenyl-, 3-methoxy-5-phenyl-2-thiophenyl, 3-hydroxy-2-thiophenyl-, 3-methylsulfonylamino-2-thiophenyl-, 3-methylsulfonylaminomethyl-2-thiophenyl-, 3-dimethylamino-2-thiophenyl-, 2,4-dimethyl-5-thiazolyl-, 2-tetrahydrothiophenyl-, 3-tetrahydrothiophenyl-, 2-tetrahydrothiophenylmethyl-, 3-tetrahydrothiphenylmethyl-, 2-furanyl-, 3-furanyl-, 2-furanylmethyl-, 3-furanylmethyl-, 2-tetrahydrofuranyl-, 3-tetrahydrofuranyl-, 2-pyridylmethyl, 3-pyridylmethyl-, 4-pyridylmethyl-, cyclopentyl, 1-cyclohexenyl-, 1-cyclopentenyl-, 1-cyclohexenylmethyl-, 1-cyclopentenylmethyl-, cyclopentylmethyl-, 4-$H_2NC_6H_4CH_2$-, 3-$H_2NC_6H_4CH_2$-, 2-$H_2N$-$C_6H_4CH_2$-, 4-$(CH_3)_2N$-$C_6H_4$-CH2-3-$(CH_3)_2N$-$C_6H_4$-$CH_2$-, 2$(CH_3)_2N$-$C_6H_4$-$CH_2$-, 4-$CH_3CONH$-$C_6H_4CH_2$-, 2-$CH_3CONHC_6H_4$-$CH_2$-, 4-$CH_3SO_2NH$-$C_6H_4CH_2$-.

9.- Compounds of formula I, wherein:

$R^1$ = phenylmethyl, 2-phenylethyl, 1-methylethyl, methyl, ethyl,

$R^2$ = H, OH

n = 0

z = CONH

in which the $R^3(CH_2)_m$ radical represents:
$C_6H_5CH=CH$-, $C_6H_5CH_2$-, 4-$CH_3COO$-$C_6H_4CH_2$, 3-$CH_3COO$-$C_6H_4CH_2$-, 2-$CH_3COO$-$C_6H_4$-$CH_2$-, 4-HO-$C_6H_4$-$CH_2$-, 3-HO-$C_6H_4$-$CH_2$-, 2-HO-$C_6H_4$-$CH_2$-, 3,4,5-$(CH_3O)$-$C_6H_2$-$CH_2CH_2$ 4-Cl-$C_6H_4$-$CH_2$-, 3-Cl-$C_6H_4CH_2$-, 2-Cl-$C_6H_4$-$CH_2$-, 4-F-$C_6H_4$-$CH_2$-, 3-F-$C_6H_4$-$CH_2$-, 2-F-$C_6H_4$-$CH_2$-, 4-HO-$C_6H_4CH_2CH_2$-, 4-$CH_3C$ OO-$C_6H_4CH_2CH_2$-4-$CH_3$-$C_6H_4$-$CH_2$-, 3-$CH_3$-$C_6H_4$-$CH_2$-, 2-$CH_3$-$C_6H_4$-$CH_2$-, 4-$CH_3O$-$C_6H_4$-$CH_2$-4-$CH_3O$-$C_6H_4$-$CH_2CH_2$-, 3-$CH_3O$-$C_6H_4CH_2$-, 2-$CH_3O$-$C_6H_4$-$CH_2$-, 2-thiophenyl-, 3-thiophenyl-, 2-(2-thiophenyl)-ethene-1-yl, 2-(3-thiophenyl)-ethene-1-yl-, 3-methyl-2-thiophenyl-, 5-methyl-2-thiophenyl-, 3-hydroxy-5-methyl-2-thiophenyl-, 3-hydroxy-5-phenyl-2-thiophenyl-, 3-methoxy-5-methyl-2-thiophenyl-, 3-methoxy-5-phenyl-2-thiophenyl, 3-hydroxy-2-thiophenyl-, 3-methylsylfonylamino-2-thiophenyl-, 3-methylsulfonylaminomethyl-2-thiophenyl-, 3-dimethylamino-2-thiophenyl-, 2,4-dimethyl-5-thiazolyl-, 2-tetrahydrothiophenyl-, 3-tetrahydrothiophenyl-, 2-tetrahydrothiophenylmethyl-, 3-tetrahydrothiophenylmethyl-, 2-furanyl-, 3-furanyl-, 2-furanylmethyl-, 3-furanylmethyl-, 2-tetrahydrofuranyl-, 3-tegrahydrofuranyl-, 2-pyridyl, 3-pyridyl, 4-pyridyl, 4-$H_2NC_6H_4CH_2$-, 3-$H_2NC_6H_4CH_2$-, 2-$H_2N$-$C_6H_4CH_2$-, 4-$(CH_3)_2N$-$C_6H_4$-CH2-3-$(CH_3)_2N$-$C_6H_4$-$CH_2$-, 2$(CH_3)_2N$-$C_6H_4$-$CH_2$-, 4-$CH_3CONH$-$C_6H_4CH_2$-, 2-$CH_3CONHC_6H_4$-$CH_2$-, 4-$CH_3SO_2NH$-$C_6H_4CH_2$-.

10.- Compounds of formula I, wherein:
$R^1$ = phenylmethyl, 2-phenylethyl, 1-methylethyl, methyl, ethyl, $R^2$ = H, OH
n = 1
z = CONH
in which the $R^3(CH_2)_m$ radical represents:
$C_6H_5CH=CH$-, $C_6H_5CH_2$-, 4-$CH_3COO$-$C_6H_4CH_2$, 3-$CH_3COO$-$C_6H_4CH_2$-, 2-$CH_3COO$-$C_6H_4$-$CH_2$-, 4-HO-$C_6H_4$-$CH_2$-, 3-HO-$C_6H_4$-$CH_2$-, 2-HO-$C_6H_4$-$CH_2$-, 3,4,5-$(CH_3O)$-$C_6H_2$-$CH_2CH_2$ 4-Cl-$C_6H_4$-$CH_2$-, 3-Cl-$C_6H_4CH_2$-, 2-Cl-$C_6H_4$-$CH_2$-, 4-F-$C_6H_4$-$CH_2$-, 3-F-$C_6H_4$-$CH_2$-, 2-F-$C_6H_4$-$CH_2$-, 4-HO-$C_6H_4CH_2CH_2$-, 4-$CH_3C$ OO-$C_6H_4CH_2CH_2$-4-$CH_3$-$C_6H_4$-$CH_2$-, 3-$CH_3$-$C_6H_4$-$CH_2$-, 2-$CH_3$-$C_6H_4$-$CH_2$-, 4-$CH_3O$-$C_6H_4$-$CH_2$-4-$CH_3O$-$C_6H_4$-$CH_2CH_2$-, 3-$CH_3O$-$C_6H_4CH_2$-, 2-$CH_3O$-$C_6H_4$-$CH_2$-, 2-thiophenyl-, 3-thiophenyl-, 2-(2-thiophenyl)-ethene-1-yl, 2-(3-thiophenyl)-ethene-1-yl-, 2-thiophenemethyl-, 3-thiophenemethyl-3-methyl-2-thiophenyl-, 5-methyl-2-thiophenyl-, 3-hydroxy-5-methyl-2-thiophenyl-, 3-hydroxy-5-phenyl-2-thiophenyl-, 3-methoxy-5-methyl-2-thiophenyl-, 3-methoxy-5-phenyl-2-thiophenyl, 3-hydroxy-2-thiophenyl-, 3-methylsulfonylamino-2-thiophenyl-, 3-methylsulfonylaminomethyl-2-thiophenyl-, 3-dimethylamino-2-thiophenyl-, 2,4-dimethyl-5-thiazolyl-, 2-tetrahydrothiophenyl-, 3-tetrahydrothiophenyl-, 2-tetrahydrothiophenylmethyl-, 3-tetrahydrothiophenylmethyl-, 2-furanyl-, 3-furanyl-, 2-furanylmethyl-, 3-furanylmethyl-, 2-tetrahydrofuranyl-, 3-tetrahydrofuranyl-, 2-pyridyl, 3-pyridyl, 4-pyridyl, 2-pyridylmethyl-, 3-pyridylmethyl-, 4-pyridylmethyl-, cyclohexyl, cyclopentyl, 1-cyclohexenyl-, 1-cyclopentenyl-, 1-cyclohexenylmethyl-1-cyclopentenylmethyl-, cyclohexylmethyl-, cyclopentylmethyl-, 4-$H_2NC_6H_4CH_2$-, 3-$H_2NC_6H_4CH_2$-, 2-$H_2N$-$C_6H_4CH_2$-, 4-$(CH_3)_2N$-$C_6H_4$-CH2-3-$(CH_3)_2N$-$C_6H_4$-$CH_2$-, 2$(CH_3)_2N$-$C_6H_4$-$CH_2$-, 4-$CH_3CONH$-$C_6H_4CH_2$-, 2-$CH_3CONHC_6H_4$-$CH_2$-, 4-$CH_3SO_2NH$-$C_6H_4CH_2$-.

11.- Compounds of formula I, wherein:
$R^1$ = phenylmethyl, 2-phenylethyl, 1-methylethyl, methyl, ethyl,
$R^2$ = H, OH
n = 2
z = CONH
in which the $R^3(CH_2)_m$ radical represents:
$C_6H_5CH=CH$-, $C_6H_5CH_2$-, 4-$CH_3COO$-$C_6H_4CH_2$, 3-$CH_3COO$-$C_6H_4CH_2$-, 2-$CH_3COO$-$C_6H_4$-$CH_2$-, 4-HO-$C_6H_4$-$CH_2$-, 3-HO-$C_6H_4$-$CH_2$-, 2-HO-$C_6H_4$-$CH_2$-, 3,4,5-$(CH_3O)$-$C_6H_2$-$CH_2CH_2$ 4-Cl-$C_6H_4$-$CH_2$-, 3-Cl-$C_6H_4CH_2$-, 2-Cl-$C_6H_4$-$CH_2$-, 4-F-$C_6H_4$-$CH_2$-, 3-F-$C_6H_4$-$CH_2$-, 2-F-$C_6H_4$-$CH_2$-, 4-HO-$C_6H_4CH_2CH_2$-, 4-$CH_3C$ OO-$C_6H_4CH_2CH_2$-4-$CH_3$-$C_6H_4$-$CH_2$-, 3-$CH_3$-$C_6H_4$-$CH_2$-, 2-$CH_3$-$C_6H_4$-$CH_2$-, 4-$CH_3O$-$C_6H_4$-$CH_2$-4-$CH_3O$-$C_6H_4$-$CH_2CH_2$-, 3-$CH_3O$-$C_6H_4CH_2$-, 2-$CH_3O$-$C_6H_4$-$CH_2$-, 2-thiophenyl-, 3-thiophenyl-, 2-(2-thiophenyl)-ethene-1-yl, 2-(3-thiophenyl)-ethene-1-yl-, 2-thiophenemethyl-, 3-thiophenemethyl-3-methyl-2-thiophenyl-, 5-methyl-2-thiophenyl-, 3-hydroxy-5-methyl-2-thiophenyl-, 3-hydroxy-5-phenyl-2-thiophenyl-, 3-methoxy-5-methyl-2-thiophenyl-, 3-methoxy-5-phenyl-2-thiophenyl, 3-hydroxy-2-thiophenyl-, 3-methylsulfonylamino-2-thiophenyl-, 3-methylsulfonylaminomethyl-2-thiophenyl-, 3-dimethylamino-2-thiophenyl-, 2,4-dimethyl-5-thiazolyl-, 2-tetrahydrothiophenyl-, 3-tetrahydrothiophenyl-, 2-tetrahydrothiophenylmethyl-, 3-tetrahydrothiophenylmethyl-, 2-furanyl-, 3-furanyl-, 2-furanylmethyl-, 3-furanylmethyl-, 2-tetrahydrofuranyl-, 3-tetrahydrofuranyl-, 2-pyridyl, 3-pyridyl, 4-pyridyl, 2-pyridylmethyl-, 3-pyridylmethyl-, 4-pyridylmethyl-, cyclohexyl, cyclopentyl, 1-cyclohexenyl-, 1-cyclopentenyl-, 1-cyclohexenylmethyl-1-cyclopentenylmethyl-, cyclohexylmethyl-, cyclopentylmethyl-, 4-$H_2NC_6H_4CH_2$-, 3-$H_2NC_6H_4CH_2$-, 2-$H_2N$-$C_6H_4CH_2$-, 4-$(CH_3)_2N$-$C_6H_4$-CH2-3-$(CH_3)_2N$-$C_6H_4$-$CH_2$-, 2$(CH_3)_2N$-$C_6H_4$-$CH_2$-, 4-$CH_3CONH$-$C_6H_4CH_2$-, 2-$CH_3CONHC_6H_4$-$CH_2$-, 4-$CH_3SO_2NH$-$C_6H_4CH_2$-.

12.- Compounds of formula I, according to Claims 6, 7, 8, 9, 10 and 11, as well as pharmaceutically acceptable salts thereof, preferably chlohydrate, bromhydrate, citrate, tartrate and maleate.

European Patent Office

# EUROPEAN SEARCH REPORT

Application Number

EP 88 50 0050

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| X | DE-A-1 925 065 (SCIENCE-UNION ET CIE) * examples 4, 8, 9, 11, 12; page 3, lines 12 - 29 * --- | 1, 3, 7, 12 | C 07 D 211/58<br>C 07 D 211/26<br>C 07 D 409/12<br>C 07 D 417/12<br>C 07 D 401/12<br>C 07 D 405/12<br>C 07 D 409/14<br>C 07 D 417/14<br>C 07 D 401/14<br>C 07 D 405/14 //<br>A 61 K 31/445<br>A 61 K 31/44 |
| X | DE-C-1 045 405 (SANDOZ AG) * examples 1, 2, 5; column 4, lines 44 - 53 * --- | 1, 3 | |
| X | US-A-4 029 801 (J.F. CAVALLA et al.) * examples 5 - 9, 12, 13, 22, 23, 27, 29 - 33, 35, 37 - 41, 46 - 49, 53, 54, 59 - 62, 81, 84, 85, 93 - 96, 100 - 102; column 1, lines 53 - column 2, line 4 * --- | 1 | |
| X | EP-A-0 229 391 (EISAI CO LTD) * examples 2, 3, 19 - 27, 31, 36 - 39, 70 - 96, 103, 104, 115 - 127, 131; page 15, lines 1 - 21 * --- | 1, 3, 8, 12 | |
| X | FR-A-2 264 530 (ANTONIO GALLARDO S.A.) * example 17d; page 31, lines 2 - 21, 24 - 35; table I, compounds 1, 2, 8, 10 - 18, 20, 21; page 5, line 39 - page 9, line 15; page 1, lines 1 - 18 * --- | 1 - 4 | **TECHNICAL FIELDS SEARCHED (Int. Cl.4)** |
| X | DE-A-2 816 884 (BEECHAM GROUP LTD) * claims 1 - 25, 30 - 33; examples 1 - 5, 8 - 11; page 22, lines 20 - 22 * --- | 1, 2, 5 | C 07 D 211/00<br>C 07 D 409/00<br>C 07 D 417/00<br>C 07 D 401/00<br>C 07 D 405/00<br>A 61 K 31/00 |
| X | BE-A- 562 880 (S.A. SANDOZ) * example 1; page 3, lines 9, 10 * --- | 1, 3 | |
| X | AT-B- 190 932 (SANDOZ AG) * complete document * --- -/- | 1, 3 | |

The present search report has been drawn up for all claims

| Place of search | Date of completion of the search | Examiner |
|---|---|---|
| BERLIN | 26-01-1989 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 88 50 0050

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-A-3 334 594 (BOEHRINGER INGELHEIM KG) * claims; example 12 * --- | 1, 3 | |
| X | US-A-3 787 419 (W.F. BRUCE) * column 5, lines 36, 37, 43; column 2, lines 27 - 33 * --- | 1, 3 | |
| X | EP-A-0 026 469 (SANDOZ AG) * page 19, example a * --- | 1, 3 | |
| X | POLISH JOURNAL OF CHEMISTRY vol. 52, no. 6, 1978, pages 1279 - 1282; B. GUTKOWSKA et al.: "Synthesis of 1-butyl-4-piperidylmethylamides of some aromatic acids" * table, compounds 1a - e, 2a, 3a - c * --- | 1, 3 | |
| X | ROCZNIKI CHEMII vol. 50, no. 1, 1976, pages 133 - 138; A. CHODKOWSKI et al.: "Synthesis of 1-butyl-4-piperidyl-and1-butyl-4-phenyl-4-piperidyl-methylamides of pyridine-carboxylic acids" * table, compounds 1a - c * --- | 1, 3 | TECHNICAL FIELDS SEARCHED (Int. Cl.4) |
| X | EP-A-0 035 374 (JOHN WEYTH & BROTHER LTD) * claims 1 - 5, 7 - 9; example 44; page 2, lines 4 - 12 * --- -/- | 1, 5 | |

The present search report has been drawn up for all claims

| Place of search | Date of completion of the search | Examiner |
|---|---|---|
| BERLIN | 26-01-1989 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 88 50 0050

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | POLISH JOURNAL OF PHARMACOLOGY AND PHARMACY vol. 32, 1980, pages 141 - 148; P. JANICKI et al.: "Analgesic Activity and Opiate Receptor Affinity of New Derivatives of N-Butylpiperidine" * page 141, compound BG - 26 * | 1 | |
| X | FR-A-2 534 255 (DELALANDE SA) * claims 1 - 4; table I; page 7, lines 1 - 6 * | 1 | |
| X | DE-A-2 747 961 (ANPHAR SA) * page 5, line 20 - page 7, line 7 * | 1 | |

TECHNICAL FIELDS SEARCHED (Int. Cl.4)

The present search report has been drawn up for all claims

| Place of search | Date of completion of the search | Examiner |
|---|---|---|
| BERLIN | 26-01-1989 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document